# EUROPÄISCHE PATENTANMELDUNG

(11) **EP 4 813 981 A1**
(43) Veröffentlichungstag der Anmeldung: **30.09.2026**
(21) Anmeldenummer: 26165415.6
(22) Anmeldetag: 17.03.2026
(51) Int. Cl.: C07D 403/06

(54) **PYRROLIDON-SUBSTITUIERTE DICARBONSÄURE, DEREN ESTER UND VERFAHREN ZU DEREN HERSTELLUNG**

(30) Priorität: 26.03.2025 DE 102025111719
(71) Anmelder: Rheinisch-Westfälische Technische Hochschule Aachen, abgekürzt RWTH Aachen, Körperschaft des öffentlichen Rechts, 52062 Aachen (DE)
(72) Erfinder: Palkovits, Regina, 52074 Aachen (DE); Sebers, Rebecca, 52070 Aachen (DE); Hausoul, Peter J.C., 6372 NR Landgraaf (NL)
(74) Vertreter: Michalski Hüttermann & Partner mbB

(57) **Zusammenfassung**

Die Erfindung betrifft ein Verfahren zur Herstellung einer Pyrrolidon-substituierten Dicarbonsäure oder deren Ester, wobei man Itaconsäure oder einen Itaconsäureester der Formel (1) mit Histidin oder einem Histidinester der Formel (2) zu einer Pyrrolidon-substituierten Dicarbonsäure oder deren Ester der Formel (3) umsetzt. Die Erfindung betrifft ferner Pyrrolidon-substituierte Dicarbonsäuren oder deren Ester der Formel (3), insbesondere 1-Carboxy-2-(1H-imidazol-4-yl)ethyl)-5-oxopyrrolidin-3-carbonsäure.

## Beschreibung

Die Erfindung betrifft Pyrrolidon-substituierte Dicarbonsäure sowie deren Ester und ein Verfahren zu deren Herstellung.

Eine allmähliche Erschöpfung der nicht erneuerbaren fossilen Ressourcen und das zunehmende Problem der Umweltverschmutzung machen es essenziell, die Abhängigkeit der chemischen Industrie von fossilen Ressourcen zu verringern und Plattformchemikalien auf Basis nachwachsender Rohstoffe zur Verfügung zu stellen, um einen Wandel zu einer nachhaltigen chemischen Industrie zu stützen.

Bedeutende Plattformchemikalien sind Verbindungen mit Pyrrolidon-Funktionalität wie auch Verbindungen mit Imidazol-Funktionalität. Bekannt sind für beide Funktionalitäten insbesondere medizinische Verwendungen. Beispielsweise beschreibt WO 2014/168522 A4 Glutarimid-Derivate und deren medizinische Verwendungen. US 7,692,028 B2 beschreibt 2-Oxo-1-pyrrolidin-Derivate und deren neuroprotektive Eigenschaften bei der Behandlung von Epilepsie. I. Cacciatore et al., European Journal of Medicinal Chemistry 2016, 108, 553-563, beschreiben neuroprotektive Eigenschaften von Prolin enthaltenden Peptiden im Bereich der Alzheimer-Behandlung. Asif, M., Alghamdi, S., Russian Journal of Organic Chemistry 2021, 57 (10), 1700-1718, geben einen Überblick über die biologische Bedeutung von Pyrrolon- und Pyrrolidinon-Derivativen.

Auch Verbindungen mit Imidazol-Funktionalität zeigen ein breites Gebiet an Anwendungsmöglichkeiten. Bekannt sind insbesondere medizinische Verwendungen. So beschreibt EP 0 442 348 B1 Imidazolverbindungen, Verfahren zu deren Herstellung, Arzneimittel auf Basis dieser Verbindungen sowie Zwischenprodukte. Alghamdi SS, et al., Drug Des Devel Ther 2021,15, 3289-3312, beschreiben Eigenschaften und Verwendungen von Imidazol. Auch Tolomeu, H.V., Fraga, C.A.M., Molecules 2023, 28, 838, beschreiben die Synthese, Funktionalisierung und Eigenschaften von Imidazol.

Ferner sind für beide Funktionalitäten auch nichtmedizinische Verwendungen bekannt. Sarala Selambakkannu et al., Radiation Physics and Chemistry, 153, 58-69 (2018) beschreiben, dass Imidazol-funktionalisierte Polymere Schwermetalle adsorbieren. Auch Bita Soleimani et al., J. Mex. Chem. Soc. 2017, 61(3), 229-240, beschreiben, dass Imidazol-basierte Polyamide und Polyimide Schwermetallionen adsorbieren. M. A. M. Alwi et al., ACS Omega 2021, 6, 25179-25192, beschreiben dass Polyvinylpolypyrrolidone Phenole in wässrigem Medium adsorbieren können. Login, R.B., JAOCS, Vol. 72, no. 7, 759-771, 1995, gibt einen Überblick über Pyrrolidon-basierte Tenside.

Die Herstellung von Pyrrolidon-Derivaten aus Itaconsäure und primären Aminen wie Aminosäuren durch Ringschluss-Reaktion ist bekannt. WO 2016/040962 A1 beschreibt die Möglichkeit der Umsetzung eines Amins, wie 2-Aminoethanol, Aminoethylethanolamin, Tris-hydroxylmethylamin, Glucosamin, Glycin, Dodecylamin oder eines anderen Monoamins, mit Itaconsäure oder Itaconsäureester. US 2019/0023839 A1 beschreibt die Umsetzung von Itaconsäure mit Glycin, Alanin, Valin, Leucin und Iso-Leucin zu Carboxyoxopyrrolidin-carboxylsäuren. CN 116874474 A beschreibt die Umsetzung von Itaconsäure mit Glycin, Alanin, Valin, Leucin, Iso-Leucin, Phenylalanin und Methionin. CN 114133557 A beschreibt die Umsetzung von Itaconsäure mit Asparaginsäure.

Die bekannten Herstellverfahren lassen Raum für Verbesserungen und es besteht Bedarf an weiteren aus Itaconsäure und einer Aminosäure herstellbaren Pyrrolidon-Derivaten.

Der vorliegenden Erfindung lag die Aufgabe zu Grunde, ein Pyrrolidon-Derivat und ein Verfahren zu dessen Herstellung aus Itaconsäure und einer Aminosäure zur Verfügung zu stellen.

Diese Aufgabe wird gelöst durch ein Verfahren zur Herstellung einer Pyrrolidon-substituierten Dicarbonsäure oder deren Ester, wobei man Itaconsäure oder einen Itaconsäureester der nachstehenden Formel (1) mit Histidin oder einem Histidinester der nachstehenden Formel (2) zu einem Imidazol-substituierten Pyrrolidon der nachstehenden Formel (3) umsetzt: 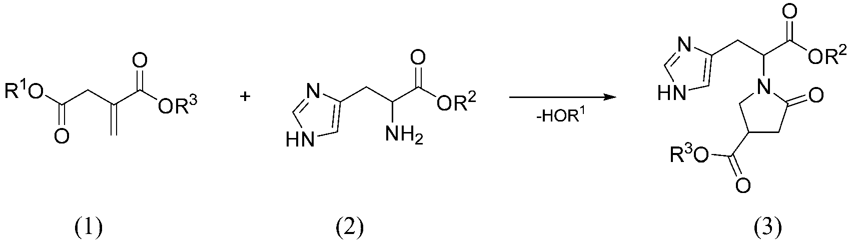 worin:
- R¹: ausgewählt ist aus der Gruppe umfassend Wasserstoff, Methyl und Ethyl,
- R²: ausgewählt ist aus der Gruppe umfassend Wasserstoff und C₁-C₄-Alkyl, und
- R³: ausgewählt ist aus der Gruppe umfassend Wasserstoff und C₁-C₄-Alkyl.

Das Verfahren erlaubt einen effizienten Zugang zu vielfach verwendbaren funktionalisierten Verbindungen. Das Verfahren erlaubt insbesondere eine nachhaltige Herstellung auf Basis Biomasse-basierter Edukte.

Der Begriff "C₁-C₄-Alkyl" umfasst, wenn nicht anders angegeben, geradkettige oder verzweigte Alkylgruppen mit 1 bis 4 Kohlenstoffatomen. C₁-C₄-Alkyl-Gruppen können ausgewählt sein aus der Gruppe umfassend Methyl, Ethyl, Propyl, Isopropyl, Butyl und Isobutyl.

R¹ ist ausgewählt aus der Gruppe umfassend Wasserstoff, Methyl und Ethyl. In Ausführungsformen ist R¹ Wasserstoff. In anderen Ausführungsformen ist R¹ Methyl oder Ethyl. Ethylester und insbesondere Methylester bilden ebenfalls gute Abgangsgruppen. R³ ist ausgewählt aus der Gruppe umfassend Wasserstoff und C₁-C₄-Alkyl. In Ausführungsformen ist R³ Wasserstoff. In anderen Ausführungsformen ist R³ Methyl oder Ethyl. In bevorzugten Ausführungsformen sind R¹ und R³ Wasserstoff. In Ausführungsformen sind R¹ und R³ unabhängig voneinander Wasserstoff, Methyl oder Ethyl, insbesondere können R¹ und R³ Methyl sein. Monoethylitaconat, Monomethylitaconat, wie auch Diethylitaconat und Dimethylitaconat sind kommerziell erhältlich.

In Ausführungsformen ist R² ausgewählt aus der Gruppe umfassend Wasserstoff, Methyl und Ethyl. In bevorzugten Ausführungsformen ist R² Wasserstoff. In Ausführungsformen ist R² Methyl oder Ethyl, insbesondere Methyl. Neben Histidin sind ebenfalls Histidinethylester und insbesondere Histidinmethylester kommerziell erhältlich.

In bevorzugten Ausführungsformen sind R¹, R² und R³ Wasserstoff. In dieser Ausführungsform ist das Verfahren eine Umsetzung von Itaconsäure mit Histidin, vorzugsweise dem natürlich vorkommenden L-Histidin. In dieser Ausführungsform kann die Herstellung aus nachwachsenden Rohstoffen auf Basis der aus Biomasse gewonnenen Substrate L-Histidin und Itaconsäure erfolgen.

Unter Verwendung von Itaconsäure und Histidin konnten hohe Ausbeuten von über 90 % bei der Umsetzung erzielt werden. Es wird vermutet, dass eine vollständige Umsetzung aufgrund der Wasserabspaltung nicht erreicht wurde, und für den Fall, dass dieses zurückgewonnen und weiterverwendet werden würde, eine Ausbeute von 100 % oder nahezu 100 % erzielbar sein sollte.

In Ausführungsformen führt man die Umsetzung in einem Lösemittel ausgewählt aus Wasser oder wässriger Lösung durch. Vorzugsweise ist das Lösemittel der Umsetzung Wasser oder eine basische oder saure wässrige Lösung. L-Histidin ist gut löslich in Wasser sowie in verdünnten Säuren und Basen. Wasser erwies sich als vielversprechendes Lösemittel. Verwendbar ist insbesondere entionisiertes oder vollentsalztes Wasser. Es wird vermutet, dass aufgrund der eingeschränkten Löslichkeit der gebildeten 1-Carboxy-2-(1H-imidazol-4-yl)ethyl)-5-oxopyrrolidin-3-carbonsäure sich Wasser als gutes Lösemittel erwies. Versuche zeigten, dass Ethanol, Methanol und Acetonitril als Lösemittel wenig geeignet waren.

In Ausführungsformen führt man die Umsetzung Katalysator-frei oder in Gegenwart eines Katalysators durch. Ohne Katalysator wurden gute Ausbeuten in einem Bereich von 55-68 % erzielt. Katalysatoren bilden eine vielversprechende Möglichkeit, Reaktionen wirtschaftlicher und ökologischer zu gestalten. Vorzugsweise führt man die Umsetzung in Gegenwart eines Alumosilikat (Zeolith)-Katalysators durch. Es konnte festgestellt werden, dass Zeolithe die Reaktion sowohl zeit- als auch temperaturabhängig deutlich effizienter machen konnten. Es wird angenommen, dass Zeolithe die Selektivität für die Bildung von 1-Carboxy-2-(1H-imidazol-4-yl)ethyl)-5-oxopyrrolidin-3-carbonsäure fördern können.

Ohne auf eine bestimmte Theorie festgelegt sein zu wollen, wird angenommen, dass der Umsetzung eine Aza-Michael-Addition zugrunde liegt. Bei der Umsetzung von Itaconsäure mit L-Histidin wird angenommen, dass ein Zwischenprodukt (S,E)-3-Carboxy-2-(((1-Carboxy-2-(1H-imidazol-4-yl)ethyl)ammonio)methyl)-1-hydroxyprop-1-en-1-olat im Gleichgewicht mit 2-((((S)-1-Carboxy-2-(1H-imidazol-4-yl)ethyl)amino)methyl)-bernsteinsäure (TABS) gebildet wird, wobei letzteres über einen Ringschluss das Produkt 1-((R)-1-Carboxy-2-(1H-imidazol-4-yl)ethyl)-5-oxopyrrolidin-3-carbonsäure (HPDA) bildet. Zeolithe haben sich hierbei als insbesondere geeignet erwiesen, den Übergang von IABS zu HPDA zu erleichtern und auch bei hohen Temperaturen stabil zu bleiben.

Unter dem Begriff "Zeolith" werden kristalline Alumosilikate mit einer offenen, dreidimensionalen Gerüststruktur aus [SiO₄]⁴⁻- und [AlO₄]⁵⁻-Tetraedern verstanden. Diese Tetraeder sind über gemeinsame Sauerstoffatome verbunden, wodurch ein poröses Netzwerk mit definierten Kanälen und Hohlräumen entsteht. Durch den Einbau von Aluminium anstelle von Silizium entsteht eine negative Ladung im Gerüst, die durch Kationen wie Alkali- und Erdalkalimetallionen (z. B. Natrium, Kalium, Magnesium oder Calcium) ausgeglichen wird. Wird ein Austausch ganz oder teilweise durch Protonen vorgenommen, entstehen Brønsted-saure-Zentren, und der Zeolith weist eine Azidität auf. Solche Materialien werden als H-Zeolithe bezeichnet. Die Azidität und Basizität eines Zeoliths können durch gezielten Ionenaustausch mit Protonen oder Metallkationen modifiziert werden. Zeolithe mit katalytisch relevanten Eigenschaften sind kommerziell erhältlich oder auf dem Fachmann bekannte Weise herstellbar.

In bevorzugten Ausführungsformen ist der Zeolith-Katalysator ausgewählt aus Mordenit-Zeolithen, insbesondere protonierten Mordenit-Zeolithen, und Zeolithen mit Beta-Struktur. Es konnte festgestellt werden, dass die Verwendung von Mordenit-Zeolithen, insbesondere protonierten Mordenit-Zeolithen, und Zeolithen mit Beta-Struktur bei gleichbleibenden Parametern an Temperatur, Reaktionszeit und Lösemitteln eine Verbesserung der Reaktionsausbeute unter den gewählten Bedingungen von etwa 10 % erzielten.

Unter dem Begriff "Mordenit-Zeolithe" (MORs) werden eine Gruppe poröser Alumosilikat-Zeolithe mit einer charakteristischen ein-dimensionalen Porenstruktur aus parallelen Kanälen verstanden. Unter dem Begriff "protonierte" Mordenit-Zeolithe werden Mordenit-Zeolithe verstanden, bei denen die ursprünglich vorhandenen, nicht ins Grundgerüst eingebauten ("extraframework'') Kationen durch Protonen ersetzt wurden. Zeolith-Katalysatoren mit Mordenit (MOR)-Strukturen sind in protonierter Form kommerziell mit verschiedenen SiO₂:Al₂O₃-Verhältnissen erhältlich, beispielsweise von der Firma Clariant mit SiO₂:Al₂O₃-Verhältnissen von 14, 20,6 und 40 unter den Bezeichnungen HCZM 14, HCZM 20,6 und HCZM 40. Ionenausgetauschte Formen lassen sich durch Austausch der Protonen gegen Metallkationen wie Lithium-, Kalium- oder Cäsiumkationen herstellen. Dies erfolgt typischerweise durch Rühren des Zeoliths in einer wässrigen Lösung der entsprechenden Metallnitrate oder -chloride. Durch die Einführung dieser Ionen werden basische Eigenschaften integriert. Die Basizität ionenausgetauschter Zeolithe steigt mit zunehmendem Ionenradius des Metallkations. Größere Kationen wie Cs⁺ schirmen die negative Ladung des Zeolithengerüsts weniger ab, wodurch die Basizität steigt. Kleinere Kationen wie Li⁺ haben eine höhere Ladungsdichte, ziehen Elektronen stärker an und verringern die Basizität. Die Basizitätsreihenfolge der Alkalimetalle in Zeolithen ist: Cs⁺ > Rb⁺ > K⁺ > Na⁺ > Li⁺.

Unter dem Begriff "Zeolithe mit Beta-Struktur" (BEAs) werden kristalline, mikroporöse Alumosilikate, die zur Gruppe der Zeolithe gehören und eine spezifische Beta-Struktur (BEA) aufweisen, verstanden. Diese Struktur ist durch ein dreidimensionales, hochgeöffnetes Porensystem mit großen Poren von ca. 6,6-7,7 Å charakterisiert, das aus miteinander verbundenen zweidimensionalen 12-gliedrigen Ringsystemen ausgebildet ist. Beta-Zeolith-Katalysatoren mit verschiedenen SiO₂:Al₂O₃-Verhältnissen sind beispielsweise von der Firma Clariant unter den Bezeichnungen HCZB 25, HCZB 30 und HCZB 150 kommerziell erhältlich.

Mordenit-Zeolithe und Beta-Zeolith weisen ähnliche Hauptporengrößen im Bereich von etwa 6,4-7,0 Å auf, unterscheiden sich jedoch in ihrer Porengeometrie. Beta-Zeolith weist ein dreidimensionales, offenes Porensystem auf, während Mordenit ein eindimensionales System mit Nebenkanälen aufweist, wodurch die Diffusionseigenschaften beeinflusst werden.

Die Zeolith-Katalysatoren können in Form von Pulvern, Granulaten oder Partikeln eingesetzt werden. Weiterhin können die Zeolithe in einer anorganischen Matrix eingebettet sein, welche bevorzugt inert ist. Geeignete anorganische Matrixmaterialien sind beispielsweise konventionelle Trägermaterialien wie Silika, Aluminiumoxid, Zirkoniumoxid, Alumosilikate, synthetische poröse Materialien oder Ton. Insbesondere keramische Träger sind bevorzugt.

In Ausführungsformen führt man die Umsetzung bei einer Temperatur im Bereich von ≥ 120 °C bis ≤ 210 °C, vorzugsweise bei einer Temperatur im Bereich von ≥ 180 °C bis ≤ 210 °C, durch. Für Umsetzungen ohne Katalysator wurden gute Ausbeuten bei Temperaturen im Bereich von ≥ 180 °C bis ≤ 210 °C erzielt. Für Umsetzungen unter Verwendung von Mordenit-Zeolithen, insbesondere protonierten Mordenit-Zeolithen, wurden gute Ausbeuten bei Temperaturen im Bereich von ≥ 150 °C bis ≤ 210 °C erzielt.

In Ausführungsformen liegt die Reaktionszeit der Umsetzung im Bereich von ≥ 3 Stunden bis ≤ 24 Stunden, bevorzugt im Bereich von ≥ 3 Stunden bis ≤ 16 Stunden. Bei geringeren oder höheren Temperaturen nahm die Ausbeute ab. Für Umsetzungen ohne Katalysator wurden gute Ausbeuten im Bereich von ≥ 3 Stunden bis ≤ 24 Stunden, bevorzugt im Bereich von ≥ 16 Stunden bis ≤ 24 Stunden erzielt. Für Umsetzungen unter Verwendung von Mordenit-Zeolithen, insbesondere protonierten Mordenit-Zeolithen, wurden gute Ausbeuten ≥ 3 Stunden bis ≤ 16 Stunden erzielt.

In bevorzugten Ausführungsformen werden äquimolare Mengen an Itaconsäure und Histidin in deionisiertem Wasser unter Verwendung eines Zeolith-Katalysators bei einer Temperatur von 180-210 °C während 3-16 Stunden umgesetzt.

Das Verfahren kann grundsätzlich kontinuierlich oder diskontinuierlich, beispielsweise in Rührkesseln, Synthesereaktoren wie Rührreaktoren oder Autoklaven durchgeführt werden. Es ist bevorzugt, dass die Umsetzung unter Stickstoffatmosphäre durchgeführt wird. Hierzu kann ein Autoklav vor der Umsetzung mit Stickstoff gespült werden.

Die aus dem Verfahren resultierenden Reaktionsgemische können nach herkömmlichen Methoden aufgereinigt werden, beispielsweise thermisch mittels destillativer oder rektifikativer Verfahren. In Ausführungsformen reinigt man die Pyrrolidon-substituierte Dicarbonsäure oder deren Ester mittels Fällung in Methanol und Waschen mit einem Gemisch von Methanol und Dimethylsulfoxid (DMSO) auf. Beispielsweise kann ein DMSO/MeOH Gemisch im Verhältnis 1:5 verwendet werden. Durch Ausfällung in Methanol aus wässriger Produktmischung und anschließender Waschung konnte das Produkt in Form eines weißen Pulvers erhalten werden.

Insgesamt kann ein Verfahren zur Verfügung gestellt werden, das unter Verwendung von gut verfügbaren, Biomasse-basierten Edukten eine Herstellung von Pyrrolidon-substituierten Dicarbonsäuren oder deren Estern in guter Ausbeute erlaubt.

Ein weiterer Gegenstand sind Pyrrolidon-substituierte Dicarbonsäuren oder deren Ester der nachstehenden Formel (3), insbesondere hergestellt nach dem hierin beschriebenen Verfahren: 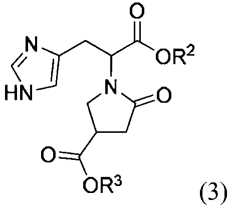 worin:
- R²: ausgewählt ist aus der Gruppe umfassend Wasserstoff und C₁-C₄-Alkyl, und
- R³: ausgewählt ist aus der Gruppe umfassend Wasserstoff und C₁-C₄-Alkyl.

In Ausführungsformen sind R² und/oder R³ unabhängig voneinander ausgewählt aus der Gruppe umfassend Wasserstoff, Methyl und Ethyl. In Ausführungsformen sind R² und/oder R³ unabhängig voneinander Methyl oder Ethyl, insbesondere Methyl. In bevorzugten Ausführungsformen sind R² und R³ Wasserstoff.

In bevorzugten Ausführungsformen ist die Pyrrolidon-substituierte Dicarbonsäure 1-Carboxy-2-(1H-imidazol-4-yl)ethyl)-5-oxopyrrolidin-3-carbonsäure, insbesondere 1-((R)-1-Carboxy-2-(1H-imidazol-4-yl)ethyl)-5-oxopyrrolidin-3-carbonsäure.

Wenn nicht anders ausgeführt, weisen die verwendeten technischen und wissenschaftlichen Ausdrücke die Bedeutung auf, wie sie gemeinhin von einem Durchschnittsfachmann in dem Gebiet, zu dem diese Erfindung gehört, verstanden wird.

Beispiele und Figuren, die der Veranschaulichung der vorliegenden Erfindung dienen, sind nachstehend angegeben.

Hierbei zeigt:
- Figur 1: die Ausbeuten Y (Mittelwert ± SD) der Umsetzungen von Itaconsäure mit L-Histidin unter Verwendung verschiedener Zeolith-Katalysatoren sowie der unkatalysierten Reaktion ((-)).
- Figur 2: die Ausbeuten Y der Umsetzung von Itaconsäure mit L-Histidin mit Katalysator (Kreise) und ohne Katalysator (ausgefüllte Punkte) bei verschiedenen Reaktionsdauern in Figur 2a) und verschiedenen Temperaturen in Figur 2b).

### Chemikalien:

Die verwendeten Edukte wurden von Sigma-Aldrich, Fluorochem, Aldrich oder aber bezogen. Lösemittel wurden von Chemsolute GmbH bezogen.

### Kernspinresonanz (NMR):

Die Reaktionsprodukte wurden mittels ¹H-NMR und mittels ¹³C-NMR mithilfe eines Bruker DPX-400 FT-NMR-Spektrometer bei 400 MHz und Raumtemperatur gemessen. Die Signale und die chemische Verschiebung in δ-Einheiten wurden auf die restlichen Protonensignale des deuterierten Lösungsmittels bezogen 1H: DMSO-d6 (δH = 2,50 ppm); DMSO-d6 (δC = 39,52 ppm). Unter Verwendung von Mesitylen als Standard wurde quantitativ analysiert.

### Beispiel 1

Umsetzung von Itaconsäure mit L-Histidin ohne Katalysator

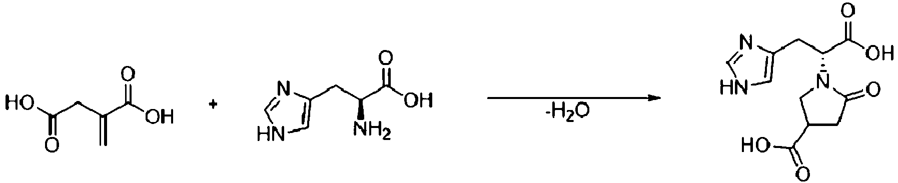

Ein Autoklav (50 mL, Eigenbau) wurde mit 0,65 mL Itaconsäure (5 mmol, 1 eq), 0,776 g L-Histidin (5 mmol, 1 eq) und 5 mL entionisiertem Wasser befüllt und anschließend drei Mal mit Stickstoff gespült. Danach wurde der Autoklav verschlossen und die Reaktion bei einer Reaktionstemperatur von 180 °C, 3 Stunden durchgeführt. Aus der erhaltenen wässrigen Produktmischung wurde in Methanol ein weißes Pulver ausgefällt, dieses wurde mit einem DMSO/MeOH (1:5) Gemisch gewaschen und getrocknet. Es konnte ein reines isoliertes Produkt 1-((R)-1-Carboxy-2-(1H-imidazol-4-yl)ethyl)-5-oxopyrrolidin-3-carbonsäure gewonnen werden.

### Beispiel 2

Untersuchung der Umsetzung von Itaconsäure mit Histidin in verschiedenen Lösemitteln

Es wurde ein Lösungsmittel-Screening mit Methanol (MeOH), Ethanol (EtOH) und Acetonitril (MeCN) als Lösungsmittel durchgeführt. Die übrigen Parameter wurden wie in Beispiel 1 beschrieben auf eine Reaktionszeit von 3 h, 180 °C, unter Stickstoff und ein äquimolares Reaktantenverhältnis festgelegt.

Während sich Itaconsäure in diesen Lösungsmitteln auflöste, zeigte L-Histidin in keinem der Lösungsmittel sichtbare Löslichkeit. Die Reaktionen wurden trotzdem durchgeführt, da eine erhöhte Löslichkeit bei erhöhten Temperaturen erwartet wurde. Nach der Reaktionsdurchführung wiesen die Produktlösungen in Methanol und Ethanol eine fast identische trübe orange Farbe auf. Die Acetonitril-Lösung hatte eine trübe gelbe Farbe mit einem dispergierten Feststoff. Nach dem Trocknen ergab die MeOH-Probe ein klebriges braunes Harz. Die EtOH-Probe hatte eine vergleichbare Farbe, war aber weniger zähflüssig. Das getrocknete Acetonitril-Produkt war eine harte gelbe Masse. Die 1H-NMR-Spektren zeigen bei allen Durchläufen wenig bis keine Produktbildung. Dies zeigt, dass das gewünschte Produkt in Methanol, Ethanol und Acetonitril als Lösungsmittel nicht in nennenswerten Mengen gebildet wurde.

### Beispiel 3

Umsetzung von Itaconsäure mit L-Histidin unter Verwendung von Zeolith-Katalysatoren

Für verschiedene Mordenit-Zeolithe und Zeolithe des Beta-Typs wurden die Ausbeuten der katalysierten Reaktion von 5 mmol Itaconsäure mit 5 mmol L-Histidin bestimmt. Die Versuche wurden durchgeführt wie unter Beispiel 1 beschrieben, wobei jeweils eine Reaktionszeit von 3 Stunden bei 180 °C, 10 Gew.-% (wt%), bezogen auf Itaconsäure, Katalysator, sowie ein äquimolares Reaktantenverhältnis und 5 mL entionisiertem Wasser als Lösungsmittel gewählt wurden.

Es wurden protonierte Mordenit-Zeolithe HCZM 14, HCZM 20,6 und HCZM 40 (Clariant) mit SiO₂:Al₂O₃-Verhältnissen von 14, 20,6 und 40 für das Screening verwendet. Ferner wurden bei drei Proben des Katalysators HCZM 40 die Protonen gegen die metallischen Gegenionen Lithium, Kalium und Cäsium ausgetauscht, um die Auswirkungen der zunehmenden Basizität zu testen. Hierzu wurde HCZM 40 in einer wässrigen Lösung der entsprechenden Nitritsalze von Lithium und Cäsium gerührt. Der Kalium-Katalysator HCZM 40 (K) wurde intern zur Verfügung gestellt. Weiter wurden die Betatyp Zeolithe HCZB 25, HCZB 30 und HCZB 150 (Clariant) getestet. Der Beta-Zeolith-Typ HCZB weist ebenso wie der Mordenit-Zeolith eine Porengröße von 7 Å auf. Die Versuche wurden als Zweifachbestimmung durchgeführt. Als Kontrolle wurde die Reaktion ohne Katalysator durchgeführt. Nach jeder Reaktion wurde der Zeolith mit einer Spritze und einem Filter entfernt. Die Ausbeuten wurden mittels quantitativer Analyse des ¹H-NMR-Spektrums bestimmt.

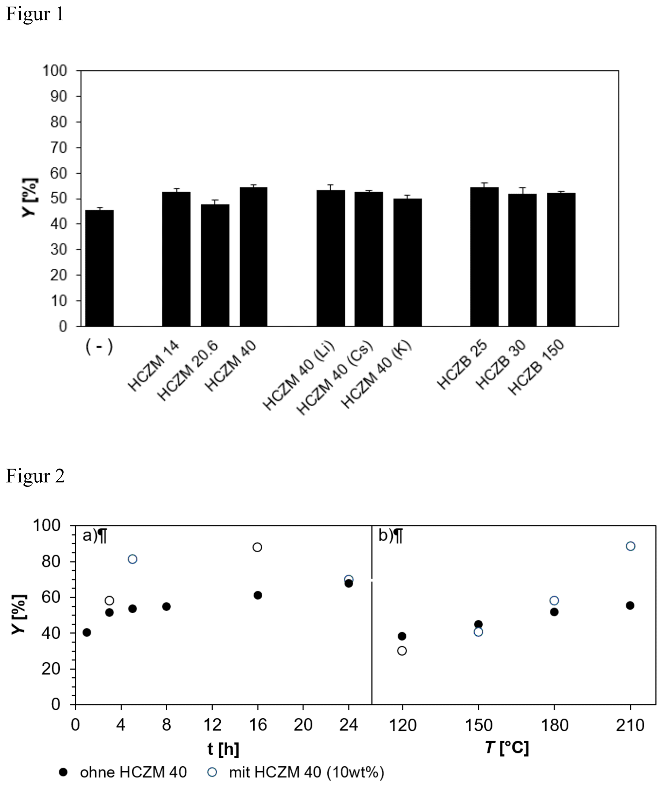

Die Ergebnisse der Ausbeuten (Mittelwert ± SD) der Umsetzungen von 5 mmol Itaconsäure mit 5 mmol L-Histidin unter Verwendung verschiedener Zeolith-Katalysatoren sowie der unkatalysierten Reaktion ((-)) sind in Figur 1 illustriert. Wie man der Figur 1 entnimmt, wurde ohne den Einsatz eines Katalysators bei den verwendeten Parametern eine Ausbeute von etwa 45 % erzielt. Unter Verwendung der Zeolith-Katalysatoren konnte die Ausbeute auf Werte zwischen 48 % und 54 % gesteigert werden, was einer maximalen Verbesserung von etwa 10 % entspricht. Diese Ergebnisse zeigen, dass die Verwendung der Katalysatoren einen positiven Einfluss auf die Reaktion hat und eine Steigerung der Reaktionsausbeute unter den gewählten Bedingungen bewirkt wurde.

### Beispiel 4

Bestimmung der Ausbeute der Umsetzung von Itaconsäure mit L-Histidin bei variierter Temperatur und Reaktionszeit

Es wurden die Auswirkungen verschiedener Reaktionszeiten sowie verschiedener Reaktionstemperaturen für die unkatalysierte Umsetzungen von Itaconsäure mit L-Histidin sowie unter Verwendung des Zeolith-Katalysators HCZM 40 (Clariant) untersucht.

Die Versuche wurden durchgeführt wie unter Beispiel 1 beschrieben, wobei jeweils ein äquimolares Reaktantenverhältnis von Itaconsäure und L-Histidin in 5 mL entionisiertem Wasser als Lösungsmittel gewählt wurde. Die katalysierten Reaktionen wurden unter Verwendung von 10 Gew.-% (wt%), bezogen auf Itaconsäure, des Katalysators HCZM 40 durchgeführt. Für die Untersuchung unterschiedlicher Reaktionszeiten wurden jeweils 5 mmol Itaconsäure mit 5 mmol L-Histidin bei 180 °C für 1, 3, 5, 8, 16 oder 24 Stunden umgesetzt.

Für die Untersuchung unterschiedlicher Reaktionstemperaturen wurden jeweils 10 mmol Itaconsäure mit 10 mmol L-Histidin für 3 Stunden bei 120 °C, 150 °C, 180 °C oder 210 °C umgesetzt.

Die Ergebnisse der Ausbeute Y in Abhängigkeit von der Reaktionszeit t bei einer konstanten Temperatur von 180 °C ist in Figur 2a) illustriert. Wie man der Figur 2a) entnimmt, stieg die Ausbeute in den ersten Stunden moderat an und erreicht ein Plateau einer Ausbeute von 70 %. Unter Verwendung des Katalysators HCZM 40 stieg die Ausbeute kontinuierlich bis 16 h an und erreichte einen maximalen Wert von 80 %. Ab Reaktionszeiten von 16 h fiel die Ausbeute unter Verwendung des Zeolithen wieder.

Die Ergebnisse der Ausbeute Y in Abhängigkeit von der Reaktionstemperatur T nach einer konstanten Reaktionszeit von 3 Stunden ist in Figur 2b) gezeigt. Wie man der Figur 2b) entnimmt, stieg die Ausbeute mit zunehmender Temperatur nur langsam an und erreichte selbst bei 210 °C nur eine Ausbeute von 55 %. Unter Verwendung des Katalysators HCZM 40 stieg die Ausbeute ab einer Temperatur von etwa 150 °C deutlich an und erreichte bei einer Temperatur von 210 °C einen maximal Wert von nahezu 90 %.

Die Untersuchung zeigt, dass die Verwendung des HCZM 40 Katalysators eine Steigerung der maximalen Ausbeute ohne Katalysator von 70 % bei 180 °C und 24 h auf eine maximale Ausbeute von 89 % sowohl bei 180 °C und 16 h wie auch bei 210 °C und 3 h erzielen konnte. Die Ergebnisse zeigen, dass der Zeolith-Katalysator HCZM 40 die Reaktion sowohl zeit- als auch temperaturabhängig deutlich effizienter machte.

Die Ergebnisse zeigen insgesamt, dass eine Herstellung von 1-((R)-1-Carboxy-2-(1H-imidazol-4-yl)ethyl)-5-oxopyrrolidin-3-carbonsäure durch Umsetzung von Itaconsäure mit L-Histidin mit guter Ausbeute zur Verfügung gestellt werden kann.

## Patentansprüche

1. Verfahren zur Herstellung einer Pyrrolidon-substituierten Dicarbonsäure oder deren Ester, wobei man Itaconsäure oder einen Itaconsäureester der nachstehenden Formel (1) mit Histidin oder einem Histidinester der nachstehenden Formel (2) zu einer Pyrrolidon-substituierten Dicarbonsäure oder deren Ester der nachstehenden Formel (3) umsetzt: 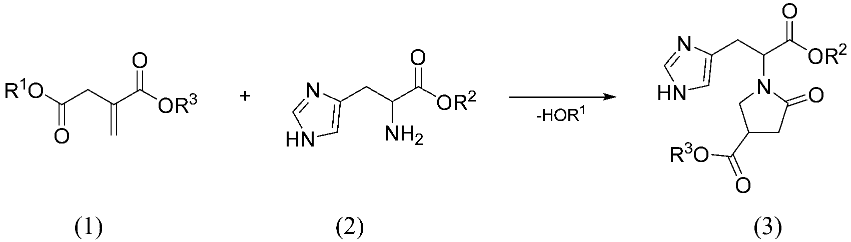 worin:
R¹ ausgewählt ist aus der Gruppe umfassend Wasserstoff, Methyl und Ethyl,
R² ausgewählt ist aus der Gruppe umfassend Wasserstoff und C₁-C₄-Alkyl, und
R³ ausgewählt ist aus der Gruppe umfassend Wasserstoff und C₁-C₄-Alkyl.

2. Verfahren nach Anspruch 1, **dadurch gekennzeichnet, dass** R² und R³ Wasserstoff sind.

3. Verfahren nach Anspruch 1 oder 2, **dadurch gekennzeichnet, dass** man die Umsetzung in einem Lösemittel ausgewählt aus Wasser oder wässriger Lösung durchführt.

4. Verfahren nach einem der vorhergehenden Ansprüche, **dadurch gekennzeichnet, dass** man die Umsetzung Katalysator-frei oder in Gegenwart eines Alumosilikat (Zeolith)-Katalysators durchführt.

5. Verfahren nach Anspruch 4, **dadurch gekennzeichnet, dass** der Zeolith-Katalysator ausgewählt ist aus Mordenit-Zeolithen und Zeolithen mit Beta-Struktur.

6. Verfahren nach einem der vorhergehenden Ansprüche, **dadurch gekennzeichnet, dass** man die Umsetzung bei einer Temperatur im Bereich von ≥ 120 °C bis ≤ 210 °C, vorzugsweise bei einer Temperatur im Bereich von ≥ 180 °C bis ≤ 210 °C, durchführt.

7. Verfahren nach einem der vorhergehenden Ansprüche, **dadurch gekennzeichnet, dass** die Reaktionszeit der Umsetzung im Bereich von ≥ 3 Stunden bis ≤ 24 Stunden, bevorzugt im Bereich von ≥ 3 Stunden bis ≤ 16 Stunden, liegt.

8. Verfahren nach einem der vorhergehenden Ansprüche, **dadurch gekennzeichnet, dass** man die Pyrrolidon-substituierte Dicarbonsäure oder deren Ester mittels Fällung in Methanol und Waschen mit einem Gemisch von Methanol und Dimethylsulfoxid aufreinigt.

9. Pyrrolidon-substituierte Dicarbonsäure oder deren Ester der nachstehenden Formel (3), insbesondere hergestellt nach einem Verfahren gemäß einem der Ansprüche 1 bis 8: 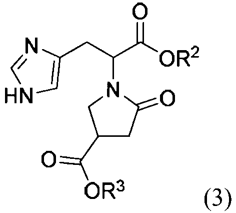 worin:
R² ausgewählt ist aus der Gruppe umfassend Wasserstoff und C₁-C₄-Alkyl, und
R³ ausgewählt ist aus der Gruppe umfassend Wasserstoff und C₁-C₄-Alkyl.

10. Pyrrolidon-substituierte Dicarbonsäure oder deren Ester nach Anspruch 9, **dadurch gekennzeichnet, dass** die Pyrrolidon-substituierte Dicarbonsäure 1-Carboxy-2-(1H-imidazol-4-yl)ethyl)-5-oxopyrrolidin-3-carbonsäure ist.
